# EUROPEAN PATENT SPECIFICATION

(11) **EP 4 051 191 B1**
(45) Date of publication and mention of the grant of the patent: **04.11.2026**
(21) Application number: 20793306.0
(22) Date of filing: 14.10.2020
(51) Int. Cl.: A61F 11/00, A61F 9/00

(54) **DRUG ADMINISTRATION DEVICE FOR EARS OR NOSE**
VORRICHTUNG ZUR VERABREICHUNG VON MEDIKAMENTEN FÜR OHREN ODER NASE
DISPOSITIF D'ADMINISTRATION DE MEDICAMENTS POUR LES OREILLES OU LE NEZ

(30) Priority: 29.10.2019 SE 1951236
(43) Date of publication of application: 07.09.2022
(73) Proprietor: OL&LI Care AB, 167 71 Bromma (SE)
(72) Inventor: LINDBLOM, Anne Elisabeth, 18333 Täby (SE); OLIVIUS, Petri, 16771 Bromma (SE)
(74) Representative: Novitas Patent AB
(86) International application number: PCT/EP2020/078899
(87) International publication number: WO 2021/083667

(56) References cited:
- WO-A1-2018/017873
- WO-A1-92/21308
- CN-A- 106 963 549
- IT-A1- UB20 160 986
- JP-A- H 021 208
- US-A1- 2014 094 759

## Description

### Field of invention

The invention relates to a medical drug administration device, and in particular to a medical drug administration device suitable for administering a drug to a human's outer ear canal.

### Background

Many people have dry skin in the outer ear canal. Such dry skin may lead to itching and irritation, which may develop into eczema.

There exist many products for cleaning or caring for the ear canal, of which cotton swabs are probably the most common. However, there are drawbacks of using cotton swabs in the ears. Itching may become worse, or even chronic. There may be a build-up of cellulose fibres in the ear. There is also a risk for damaging the ear drum, or packing ear wax towards it, which may lead to pain and reduced hearing. Moreover, use of disposable cotton swabs produces unnecessary environmental waste.

As an alternative to cotton swabs, some people use their fingers to mitigate itching. This also risks damaging the ear canal, in addition to the problem of poor access of fingers in the ear canal.

Furthermore, there is a common problem of administering ear drops or ointment to the ear canal. It is often difficult to administer the right amount of ear drops, and to get the ear drops to actually enter the ear canal. This problem is even more pronounced for treatment with ointments. Treating the ears is a problem for everyone but may be more pronounced for the elderly, of which many are living alone.

US 2014/094 759 discloses a medical drug administration device for e.g. administration of liquid drug or ointment to a person's ear canal, said device comprising a central handle piece, and a drug administration piece with an extension element extending from the central handle piece, said drug administration piece having a length in the interval of from 14 to 20 mm, wherein the drug administration piece is on its outer part, after the extension element, provided with a cup for administration of liquid drug.

The device of US 2014/094 759 was developed for usage in the eye. Said device is suitable to use on a relatively flat surface as the eye

There is still a need for a new device for cleaning the ear and treating ear canal problems.

### Short description of the figures

Fig. 1 is a perspective view of the drug administration device showing the device reaching into the ear canal.
Fig. 2 is a perspective view of the drug administration device showing the device being used for administration of eye drops.
Fig. 3 is a perspective view of the drug administration device having two drug administration parts.
Fig. 4 is a side view of the drug administration device having two drug administration parts.
Fig. 5 is a view from above of the drug administration device having only one drug administration part.

### Description of the invention

The invention relates to a medical drug administration device (1) suitable for administration of liquid drug or ointment to a person's ear canal, or nose, comprising
a central handle piece (2), and
at least one drug administration piece (3) with an extension element (4) extending from the central handle piece, wherein the drug administration piece is on its outer part, after the extension element, provided with a cup (6) for administration of liquid drug,
characterized in that the drug administration piece has a length in the interval of from 16 to 18 mm, and
wherein the drug administration piece has rounded edges (5) not to damage or irritate tissues of the ear or nose.

As an alternative, the device may be provided with two drug administration pieces (3), each having a cup (6) for administration of liquid drug. Preferably, said two drug administration pieces (3) are located opposite to one another.

The drug administration piece(s) of the device have rounded edges (5) not to damage or irritate tissues of the ear.

When the medical drug administration device has only one drug administration piece, the size of the cup corresponds to a volume of 1-2 drops or 4 drops.

When the medical drug administration device has two drug administration pieces, the size of the cups corresponds to a volume of 1-2 drops and 4 drops, respectively.

The volume(s) have been chosen based on recommended doses of ear drops.

In use, for the ear, a cup may be filled with liquid drug, the device inserted into the ear canal, and the device tilted for the liquid drug to be poured out. In the case of ointment administration, the back side of the administration piece may be covered with ointment whereupon the device is inserted into the ear canal and the ointment smeared on the relevant patch.

Ointments have an advantage in relation to ear drops in that they stay in place in the ear canal, as is often not the case with ear drops. Despite their clear advantages, ointments for ear use have not been prescribed as widely as ear drops, since there has not been provided any solution to the administration problem. With the invention as claimed, this is no longer the case.

The back side of the administration piece may moreover be used to relieve itching in the ears, by the device being turned around while the administration piece is placed in the ear.

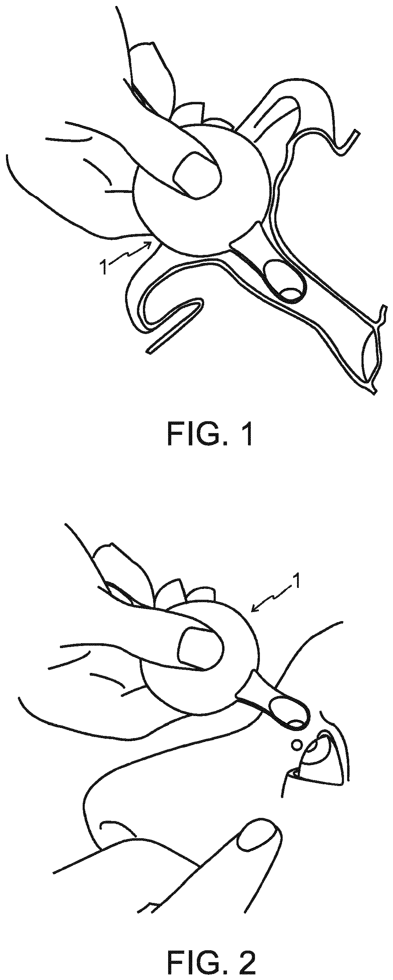

The handle piece, being of a size such that it cannot enter the ear canal, ensures that the drug administration piece cannot enter into the bony ear canal, which would lead to the ear canal skin getting compromised. This is an important safety aspect of the invention (see Fig. 1).

In use, for the eye, a cup of a device, which is not part of the invention, is filled with ear drops and emptied between the lower eyelid and the eye (see Fig. 2). Just as for ear treatment, ointment may be covered on the backside of the drug administration piece for subsequent smearing of e.g. the eyelids.

In use, for the nose, ointment may be covered on the backside of the drug administration piece for subsequent smearing of the nose mucous membrane.

Due to the device being intended for use several times, it abolishes the need for single-use pipettes, many of which being packaged in disposable packaging. This makes life easier for e.g. elderly people who may have difficulties opening disposable packaging.

The device may in its entirety be made of a non-shatter material, such as polypropylene. Polypropylene is a recyclable plastic having lower environmental impact. The fact that the device is not disposable but intended to be used several times further reduces its environmental impact. The device as claimed may be washed in water and common detergent, should the need arise.

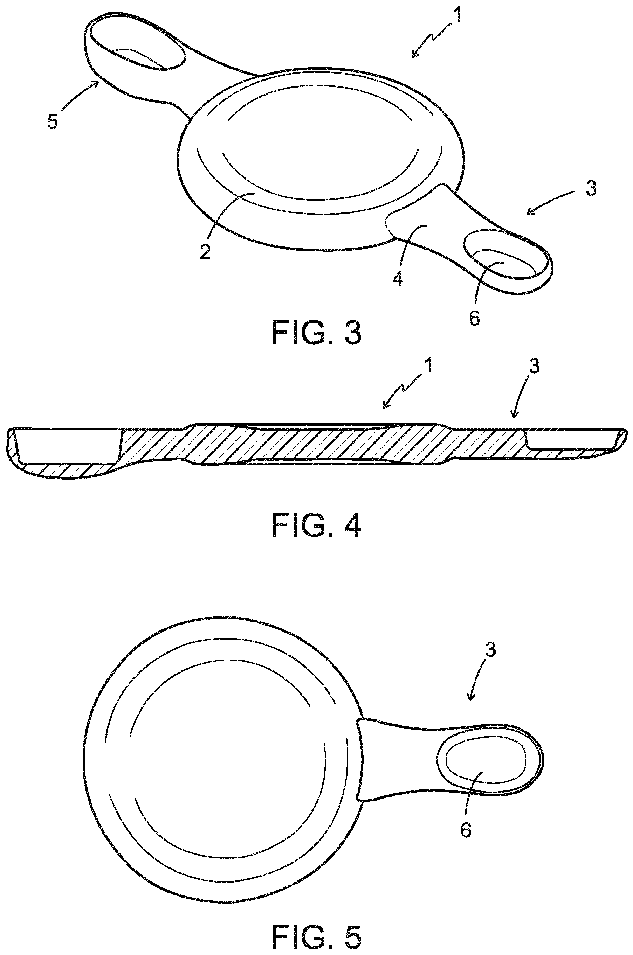

The central handle piece of the medical drug administration device may have various forms, such as round, square, hexagonal, octagonal, etc, all with a flat surface. Of these forms, a round flat form is preferred (see Fig. 3). The handle piece may have a striated surface, to facilitate handling.

The central handle piece may have a circumference in the interval of from 20 to 50 mm, e.g. 25 to 40 mm, e.g. 28 mm.

The drug administration piece(s) each, including the extension element, have a length in the interval of from 16 to 18 mm. Most preferred, each drug administration piece has a length of 17 mm. The lengths have been chosen for the device not to enter into the bony ear canal. A length of 17 mm has been found to be appropriate for most people. The stated lengths have in all cases been counted from the tip of the drug administration piece to the position where the extension element joins the central handle piece.

The width of the drug administration piece(s) is in the interval of from 5 to 9 mm, e.g. 6 to 8 mm. A width of 7 mm is preferred.

The height of the drug administration piece(s) is in the interval of from 2 to 8 mm, e.g. 3 to 7 mm, e.g. 4 to 6 mm. A preferred height is 3 or 5 mm. When the device is provided with two drug administration pieces, the cups preferably have different sizes (i.e. hold different maximum volume; see Fig. 3). A preferred combination is a device wherein one cup has a height of 3 mm, whereas the other cup has a height of 5 mm.

To summarize, the invention as claimed has many advantages. It enables clear measurement of the number of drops to be administered, provides a safe mode of administration without the risk for damaging the ear canal, and facilitates administration of ointment to e.g. the ear canal or the nose. Moreover, the drug administration device as claimed replaces disposable pipettes and cotton swabs for medication and ear canal treatment, respectively, hence reducing the need for disposable medical devices. Thus, the device as claimed reduces costs and is environmentally friendly.

The device may be provided with the manufacturer's name or slogan, preferably in relief. The drug delivery device may be packaged in a cellulose packaging material. The device may be packaged in a kit together with an ointment or liquid drug for e.g. treatment of the ear canal. The ointment may be based on beeswax, and the liquid drug may be oil based.

The kit may moreover comprise instructions for use.

Use of the device as herein described for administration of liquid drug or ointment to a person's ear canal, or nose, is as described above.

It should be realized that specific examples and embodiments provided herein are merely intended to exemplify the invention and shall not be seen as any limitations whatsoever to the scope of protection.

## Claims

1. Medical drug administration device (1) suitable for administration of liquid drug or ointment to a person's ear canal, or nose, comprising
a central handle piece (2), and
at least one drug administration piece (3) with an extension element (4) extending from the central handle piece, wherein the drug administration piece is on its outer part, after the extension element, provided with a cup (6) for administration of liquid drug,
**characterized in that** the drug administration piece has a length in the interval of from 16 to 18 mm, and
wherein the drug administration piece has rounded edges (5) adapted not to damage or irritate tissues of the ear or nose.

2. Medical drug administration device according to claim 1, wherein it is provided with two drug administration pieces (3), each having a cup (6) for administration of liquid drug.

3. Medical drug administration device according to claim 2, wherein the two drug administration pieces (3) are located opposite to one another.

4. Medical drug administration device according to claim 1, wherein the size of the cup corresponds to a volume of 1-2 drops or 4 drops.

5. Medical drug administration device according to claims 2 or 3, wherein the size of the cup corresponds to a volume of 1-2 drops and 4 drops, respectively.

6. Medical drug administration device according to any of the preceding claims, wherein the drug administration piece(s) each have a width of 5-9 mm.

7. Medical drug administration device according to any of the preceding claims, wherein it is manufactured from polypropylene.

8. Kit of parts, comprising a device according to any of the preceding claims and an ointment or liquid drug.

9. Kit of parts according to claim 8, wherein the ointment is based on beeswax, or the liquid drug is oil based.

10. Medical drug administration device according to any of claims 1- 7 or kit of parts according to any of claims 8 - 9, wherein it also comprises instructions for use.

## Patentansprüche

1. Medizinische Vorrichtung (1) zur Verabreichung von Medikamenten, geeignet zur Verabreichung von flüssigem Medikament oder Salbe in den Gehörgang oder die Nase einer Person, umfassend
ein zentrales Griffstück (2), und
mindestens ein Medikamentenverabreichungsstück (3) mit einem Verlängerungselement (4), das sich von dem zentralen Griffstück aus erstreckt, wobei das Medikamentenverabreichungsstück an seinem äußeren Teil, nach dem Verlängerungselement, mit einem Näpfchen (6) zur Verabreichung von flüssigem Medikament versehen ist,
**dadurch gekennzeichnet, dass** das Medikamentenverabreichungsstück eine Länge im Intervall von 16 bis 18 mm aufweist, und
wobei das Medikamentenverabreichungsstück abgerundete Kanten (5) aufweist, um Gewebe des Ohres oder der Nase nicht zu beschädigen oder zu reizen.

2. Medizinische Vorrichtung zur Verabreichung von Medikamenten nach Anspruch 1, **dadurch gekennzeichnet, dass** sie mit zwei Medikamentenverabreichungsstücken (3) versehen ist, die jeweils ein Näpfchen (6) zur Verabreichung von flüssigem Medikament aufweisen.

3. Medizinische Vorrichtung zur Verabreichung von Medikamenten nach Anspruch 2, **dadurch gekennzeichnet, dass** die beiden Medikamentenverabreichungsstücke (3) einander gegenüberliegend angeordnet sind.

4. Medizinische Vorrichtung zur Verabreichung von Medikamenten nach Anspruch 1, wobei die Größe des Näpfchens einem Volumen von 1-2 Tropfen oder 4 Tropfen entspricht.

5. Medizinische Vorrichtung zur Verabreichung von Medikamenten nach Anspruch 2 oder 3, wobei die Größe des Näpfchens einem Volumen von 1-2 Tropfen bzw. 4 Tropfen entspricht.

6. Medizinische Vorrichtung zur Verabreichung von Medikamenten nach einem der vorhergehenden Ansprüche, **dadurch gekennzeichnet, dass** jedes Medikamentenverabreichungsstück eine Breite von 5-9 mm aufweist.

7. Medizinische Vorrichtung zur Verabreichung von Medikamenten nach einem der vorhergehenden Ansprüche, **dadurch gekennzeichnet, dass** sie aus Polypropylen hergestellt ist.

8. Kit von Teilen, umfassend eine Vorrichtung nach einem der vorhergehenden Ansprüche und eine Salbe oder ein flüssiges Medikament.

9. Kit von Teilen nach Anspruch 8, wobei die Salbe auf Bienenwachs basiert oder das flüssige Medikament ölbasiert ist.

10. Medizinische Vorrichtung zur Verabreichung von Medikamenten nach einem der Ansprüche 1 bis 7 oder Kit von Teilen nach einem der Ansprüche 8 bis 9, **dadurch gekennzeichnet, dass** sie bzw. es auch eine Gebrauchsanweisung umfasst.

## Revendications

1. Dispositif médical d'administration de médicaments (1) apte à l'administration d'un médicament liquide ou d'une pommade dans le conduit auditif, ou le nez, d'une personne, comprenant
une pièce formant poignée centrale (2), et
au moins une pièce d'administration de médicament (3) avec un élément d'extension (4) s'étendant depuis la pièce formant poignée centrale, la pièce d'administration de médicament étant pourvue, sur sa partie extérieure, après l'élément d'extension, d'une coupelle (6) pour l'administration d'un médicament liquide,
**caractérisé en ce que** la pièce d'administration de médicament présente une longueur dans l'intervalle de 16 à 18 mm, et
**en ce que** la pièce d'administration de médicament présente des bords arrondis (5) pour ne pas endommager ou irriter les tissus de l'oreille ou du nez.

2. Dispositif médical d'administration de médicaments selon la revendication 1, **caractérisé en ce qu'**il est pourvu de deux pièces d'administration de médicament (3), ayant chacune une coupelle (6) pour l'administration d'un médicament liquide.

3. Dispositif médical d'administration de médicaments selon la revendication 2, **caractérisé en ce que** les deux pièces d'administration de médicament (3) sont situées à l'opposé l'une de l'autre.

4. Dispositif médical d'administration de médicaments selon la revendication 1, dans lequel la taille de la coupelle correspond à un volume de 1-2 gouttes ou 4 gouttes.

5. Dispositif médical d'administration de médicaments selon les revendications 2 ou 3, dans lequel la taille de la coupelle correspond à un volume de 1-2 gouttes et 4 gouttes, respectivement.

6. Dispositif médical d'administration de médicaments selon l'une quelconque des revendications précédentes, **caractérisé en ce que** la ou les pièces d'administration de médicament présentent chacune une largeur de 5-9 mm.

7. Dispositif médical d'administration de médicaments selon l'une quelconque des revendications précédentes, **caractérisé en ce qu'**il est fabriqué en polypropylène.

8. Kit de pièces, comprenant un dispositif selon l'une quelconque des revendications précédentes et une pommade ou un médicament liquide.

9. Kit de pièces selon la revendication 8, dans lequel la pommade est à base de cire d'abeille, ou le médicament liquide est à base d'huile.

10. Dispositif médical d'administration de médicaments selon l'une quelconque des revendications 1 à 7 ou kit de pièces selon l'une quelconque des revendications 8 à 9, **caractérisé en ce qu'**il comprend également des instructions d'utilisation.
